# NEUE EUROPÄISCHE PATENTSCHRIFT

(11) **EP 1 747 790 B2**
(45) Veröffentlichungstag und Bekanntmachung des Hinweises auf die Entscheidung über den Einspruch: **04.08.2010**
(45) Hinweis auf die Patenterteilung: 12.09.2007
(21) Anmeldenummer: 06009127.9
(22) Anmeldetag: 03.05.2006
(51) Int. Cl.: A61M 5/44

(54) **Vorrichtung zum Temperieren einer Fluidleitung**
Device for tempering a tube containing fluid
Dispositif pour tempérer un conduit contenant un fluide

(30) Priorität: 29.07.2005 DE 102005036369
(43) Veröffentlichungstag der Anmeldung: 31.01.2007
(73) Patentinhaber: Barkey GmbH & Co. KG, 33818 Leopoldshöhe (DE)
(72) Erfinder: Barkey, Volker, 33619 Bielefeld (DE)
(74) Vertreter: Ostermann, Thomas

(56) Entgegenhaltungen:
- DE-A1- 4 444 180
- DE-U1- 9 318 886
- DE-U1- 20 213 225
- DE-U1- 29 917 247
- GM- - 000 023 130
- US- - 6 608 968
- US-A- 5 601 894
- US-A1- 2002 156 451

## Beschreibung

Die Erfindung betrifft eine Vorrichtung zum Temperieren einer Fluidleitung nach dem Oberbegriff der Anspruchs 1.

Aus der DE 44 44 180 C2 ist eine Vorrichtung zum Temperieren einer Fluidleitung mit einer Mantelleitung bekannt, die über einen zylinderförmigen Hohlraum verfügt. Ferner weist die Mantelleitung einen Längsschlitz auf, so dass die Fluidleitung in den Hohlraum der Mantelleitung einsetzbar ist. In der eingesetzten Stellung der Fluidleitung ist dieselbe fast vollständig von der Mantelleitung umschlossen. Mittels in der Mantelleitung integrierter Heizelemente kann eine Temperierung der Fluidleitung erfolgen.

Um die Temperatur des in der Fluidleitung geführten Fluids zu regeln, ist unmittelbar am Einlass und am Auslass der Mantelleitung jeweils ein Temperatursensor angeordnet, der mit einer Regeleinheit in Verbindung steht. Die bekannte vorrichtung, die sich in der Praxis bewährt hat, weist jedoch den Nachteil auf, dass die Temperatursensoren mittels eines zusätzlichen in die Mantelleitung einsetzbaren Anschlussstückes in der Mantelleitung integriert sind. Die Temperatursensoren werden daher erst bei Anschluss der Mantelleitung an die Regeleinheit in die Mantelleitung eingeführt.

Ein Vorrichtung zum Temperieren einer Fluidleitung gemäss dem Oberbegriff von Anspruch 1 ist aus dem Dokument DE 93 18 886 U1 bekannt.

Aufgabe der vorliegenden Erfindung ist es daher, eine Vorrichtung zum Temperieren einer Fluidleitung derart weiterzubilden, dass die Wärmeeinleitung von einer die Fluidleitung umgebenden Mantelleitung in die Fluidleitung verbessert wird.

Zur Lösung dieser Aufgabe weist die Erfindung die Merkmale der Patentanspruchs 1 auf.

Der besondere Vorteil der Erfindung besteht darin, dass durch die symmetrische Anordnung einer Mehrzahl von Längskanälen zur Aufnahme von Heizelementen bzw. Temperatursensoren eine homogene Erwärmung des in der Fluidleitung geführten Fluids in radialer Richtung der Mantelleitung erfolgen kann. Die Fluidleitung kann in Umfangsrichtung gleichmäßig erwärmt werden. Insbesondere bei relativ kurzen Fluidleitungen kann hierdurch eine verbesserte Temperierung des Fluids am Ausgang der Fluidleitung erfolgen.

Nach einer bevorzugten Ausführungsform der Erfindung verläuft zwischen zwei Längskanälen zur Aufnahme von Heizelementen ein Hohlkanal, in dem mindestens ein Temperatursensor und mindestens eine den Temperatursensor mit einer Regeleinheit verbindenden Kontaktierleitung angeordnet sind. Dem Hohlkanal kommt eine Doppelfunktion zu. Zum einen dient er zur Aufnahme des Temperatursensors bzw. der Kontaktierleitung. Zum anderen kann die relativ zum Material der Mantelleitung erhöhte Temperaturleitfähigkeit der den Hohlkanal ausfüllenden Luft dazu genutzt werden, eine Homogenisierung des Wärmeeintrages entlang des Umfanges der Fluidleitung zu begünstigen. Vorteilhaft kann hierdurch die Zahl der Heizelemente begrenzt werden. Der luftgefüllte Hohlkanal kann zugleich als Außenisolierung für die Mantelleitung dienen.

Nach einer Weiterbildung der Erfindung ist der Hohlkanal zur Aufnahme des Temperatursensors im Querschnitt als ein Langloch ausgebildet, das sich zwischen zwei gegenüberliegenden Heizelementen eines umfangsabschnittes der Mantelleitung erstreckt. Die Mantelleitung weist somit entlang ihrer Umfangsabschnitte in einem vorgegeben radialen Abstand sowohl Heizelemente als auch Luft führende Hohlkanäle auf.

Nach einer Weiterbildung der Erfindung weist die Mantelleitung einen ersten und einen zweiten Umfangsabschnitt auf, die jeweils nierenförmig angeordnet sind. In einem Verbindungsbereich zwischen dem ersten und dem zweiten Umfangsabschnitt ist eine Längskerbe vorgesehen, die die Flexibilität der vorzugsweise als Mantelschlauch ausgebildeten Mantelleitung erhöht.

Ausführungsbeispiele der Erfindung werden nachfolgend anhand der Zeichnungen näher erläutert.

Es zeigen:
- Figur 1: eine perspektivische Darstellung einer Mantelleitung mit einem endseitigen Kopfelement und einer endseitigen Endkappe in Explosionsdarstellung nach einer ersten Ausführungsform,
- Figur 2: einen Querschnitt durch die Mantelleitung gemäß Figur 1 und
- Figur 3: einen Querschnitt durch eine Mantelleitung nach einer zweiten Ausführungsform.

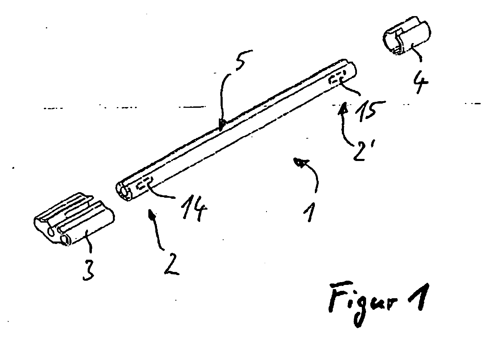

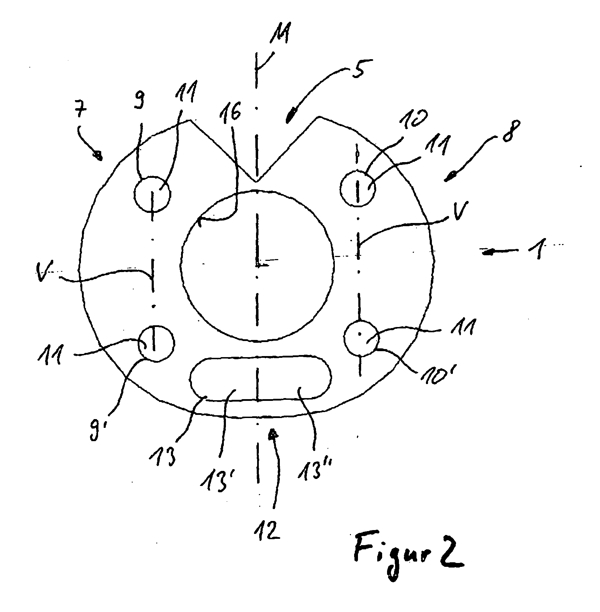

In den Figuren 1 und 2 ist eine Mantelleitung 1 nach einer ersten Ausführungsform dargestellt, die mittels eines an einem ersten Ende 2 desselben befestigten Kopfelementes 3 über ein nicht dargestelltes elektrisches Kabel an eine Regeleinheit gekoppelt ist. Darüber hinaus ist die Mantelleitung 1 über das Kopfelement 3 an einer Haltevorrichtung befestigt, die zur Halterung eines ein Fluid enthaltenden Fluidbehälters dient. An dem Fluidbehälter ist eine Fluidleitung angeschlossen, die das Fluid zum Empfänger führt. An einem dem Empfänger zugewandten Ende 2' ist die Mantelleitung 1 mit einer abschließenden Endkappe 4 versehen.

Alternativ kann die erfindungsgemäße Vorrichtung im Zusammenhang mit Dialysesystemen eingesetzt werden.

Die Mantelleitung 1 besteht aus einem flexiblen Kunststoffmaterial und ist im Querschnitt kreisringförmig ausgebildet. Die Mantelleitung 1 weist einen Längsschlitz 5 auf, durch den die Fluidleitung in einen zylinderförmigen Hohlraum 6 der Mantelleitung 1 einsetzbar oder einlegbar ist. In der Temperierstellung der Fluidleitung ist die Fluidleitung fast vollständig von einen ersten Umfangsabschnitt 7 und einem zweiten Umfangsabschnitt 8 der Mantelleitung 1 umgeben.

Wie besser aus Figur 2 ersichtlich ist, erstrecken sich der Umfangsabschnitt 7 und der zweite Umfangsabschnitt 8 zu beiden Seiten einer Längsmittelebene M der Mantelleitung 1, die durch den Längsschlitz 5 verläuft. Der erste Umfangsabschnitt 7 und der zweite Umfangsabschnitt 8 weisen jeweils zwei durchgehende Längskanäle 9, 9' bzw. 10, 10' auf, in denen sich zylinderförmige Heizelemente 11 erstrecken. Die Heizelemente 11 sind als Heizdrähte oder Heizgflecht ausgebildet, die als elektrische Leiter elektrisch mit der nicht dargestellten Regeleinheit verbunden sind. Hierzu weist die Endkappe 4 bzw. das Kopfelement 3 nicht dargestellte Umfangsbrücken auf, so dass zur Temperierung ein elektrischer Strom vorgegebener Höhe über das mehradrige Zuleitungskabel in das Kopfelement 3 eingespeist werden kann.

Die Heizdrähte 11 sind in einem gleichen radialen Abstand um den Hohlraum 6 herum angeordnet. Eine gedachte Verbindungsfläche V zwischen den Heizelementen 11 des Umfangsabschnitts 7 bzw. des zweiten Umfangsabschnitts 8 verläuft parallel zu der Längsmittelebene M. Die Heizdrähte 11 werden als Einlegeteile in ein Extrusionsformwerkzeug eingesetzt, so dass beim Strangpressen der Mantelleitung 1 eine dichte und feste Umschließung der Heizdrähte 11 durch das Kunststoffmaterial der Mantelleitung 1 gegeben ist.

In einem Verbindungsbereich 12 zwischen dem ersten Umfangsabschnitt 7 und dem zweiten Umfangsabschnitt 8 der Mantelleitung 1 erstreckt sich ein durchgehender Hohlkanal 13, in dem ersten Endbereich 2 ein Temperatursensor 14 zur Ermittlung der Temperatur am Eingang der Mantelleitung 1 und in dem zweiten Endbereich 2' ein zweiter Temperatursensor 15 zur Ermittlung der Temperatur in einem Ausgangsbereich der Mantelleitung 1 angeordnet sind. Die Temperatursensoren 14, 15 sind stoffschlüssig oder formschlüssig in dem ersten Endbereich 2 bzw. zweiten Endbereich 2' angeordnet. Von den Temperatursensoren 14, 15 verlaufen jeweils Kontaktierleitungen durch den Hohlkanal 13 zu dem Kopfelement 3, von dem aus sie über das Zuleitungskabel an die Regeleinheit weitergeführt sind. Die durch die Temperatursensoren 14, 15 ermittelten Temperaturen im Eingangs- und Ausgangsbereich der Mantelleitung 1 wird in einer Regelschaltung der Regeleinheit die elektrische Stromstärke für die Speisung der Heizdrähte 11 oder -geflechte oder - elemente ermittelt, damit das Fluid entsprechend einer vorgegebenen Sollwerttemperatur von beispielsweise 37°C dem Empfänger zur verfügung gestellt werden kann. Zur Regelung der Solltemperatur bzw. zur Einstellung der elektrischen Stromstärke als Stellgröße werden die Temperaturen des Eingangstemperatursensors 14 und des Ausgangstemperatursensors 15 berücksichtigt, wobei bereits eine Änderung der durch den ersten Temperatursensor 14 ermittelten Eingangstemperatur des Fluids eine Änderung der Stellgröße im Sinne einer kontinuierlichen Nachführung der aktuellen Fluidtemperatur an die Solltemperatur erfolgt.

Der Hohlkanal 13 ist symmetrisch zu der Längsmittelebene M angeordnet und ist im Querschnitt als ein Langloch ausgebildet. Eine erste Hälfte des Hohlkanals 13' ist dem ersten Umfangsabschnitt 7 und eine zweite Hälfte des Hohlkanals 13'' ist dem zweiten Umfangsabschnitt 8 zugehörig. Der Hohlkanal 13 weist einen zu den Heizkanälen 11 relativ großen Querschnitt auf, der im Wesentlichen durch Luft ausgefüllt ist. Der Hohlkanal 13 kann somit, da die Temperaturleitfähigkeit größer ist als die des die Mantelleitung 1 bildenden Kunststoffmaterials, zur homogenen Wärmeeinleitung an einer Wandung 16 des Hohlraums 6 dienen.

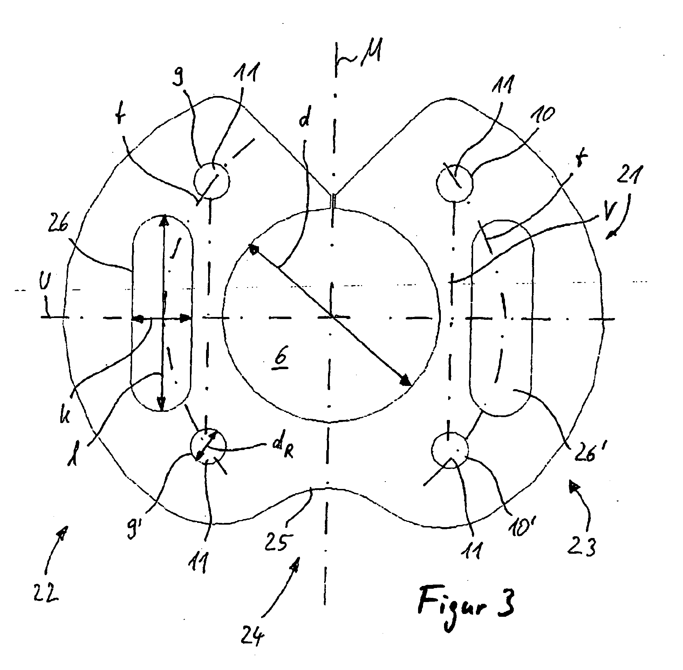

Nach einer zweiten Ausführungsform der Erfindung gemäß Figur 3 ist eine Mantelleitung 21 mit einem ersten Umfangsabschnitt 22 und einem zweiten Umfangsabschnitt 23 vorgesehen, die jeweils im Querschnitt nierenförmig ausgebildet sind. In einem Verbindungsbereich 24 zwischen dem ersten Umfangsabschnitt 22 und dem zweiten Umfangsabschnitt 23 ist außenseitig eine Längskerbe 25 vorgesehen, mittels derer die Flexibilität der Mantelleitung 21 insbesondere beim Einsetzen der Fluidleitung in den Hohlraum 6 der Mantelleitung 21 bzw. beim Entfernen der Fluidleitung aus dem Hohlraum 6 der Mantelleitung 21 verbessert wird. Gleiche Bauteile oder Funktionen der ersten Ausführungsform und der zweiten Ausführungsform sind mit den gleichen Bezugszeichen versehen.

Wie nach dem ersten Ausführungsbeispiel weisen der erste Umfangsabschnitt 22 und der zweite Umfangsabschnitt 23 jeweils zwei Heizleitungen 11 auf, deren gedachte Verbindungsfläche V parallel zu der Längsmittelebene M der Mantelleitung 21 verläuft. In einem mittleren Bereich des ersten Umfangsabschnitts 22 und des zweiten UmfangsabSchnitts 23 zwischen den Heizkanälen 11 erstreckt sich jeweils ein Hohlkanal 26, 26', wobei in dem einen Hohlkanal 26 der erste Temperatursensor und in dem zweiten Hohlkanal 26' der zweite Temperatursensor angeordnet ist. Die Temperatursensoren sind jeweils in einem gegenüberliegenden Endbereich der Mantelleitung 21, angeordnet. Die Hohlkanäle 26, 26' sowie die Längskanäle 9, 9' bzw. 10, 10' des ersten Umfangsabschnitts 22 und des zweiten Umfangsabschnitts 23 erstrecken sich symmetrisch zu der Längsmittelebene M. Die Längskanäle 9, 9', 10, 10' bzw, Hohlkanäle 26, 26' erstrecken sich im Wesentlichen entlang eines Teilkreises t der Umfangsabschnitte 22, 23. Hierdurch wird eine homogene Wärmeeinleitung in die Fluidleitung ermöglicht.

Die Hohlkanäle 26, 26' bzw. die Längskanäle 9, 9', 10, 10' sind in dem ersten Umfangsabschnitt 22 und im zweiten Umfangsabschnitt 23 derart angeordnet, dass sie symmetrisch zu einer Längsmittelebene u des ersten Umfangsabschnitts 22 und des zweiten Umfangsabschnitts 23 verlaufen. Die Mittelebene U steht senkrecht zu der Längsmittelebene M der Mantelleitung 21.

Der Hohlkanal 26, 26' weist eine lange Quererstreckung 1 auf, die gleich oder größer ist als der Durchmesser d des Hohlraums 6 der Mantelleitung 21. Der Hohlkanal 26, 26' weist eine kurze Quererstreckung k auf, deren Länge im Wesentlichen dem Durchmesser d_{R} des Längskanals 9, 9', 10, 10' entspricht.

Alternativ kann der Hohlkanal 26, 26' auch bogenförmig ausgebildet sein, wobei sich die Quererstreckung d auf dem Teilkreis t erstreckt.

Durch das Einlegen oder Einsetzen der Fluidleitung in den Hohlraum 6 der Mantelleitung 1 wird eine konduktive und konvektive Wärmeübertragung von den Heizelementen 11 zu dem die Mantelleitung 1 am stromabwärts gelegenen Ende derselben verlassenden Fluid gewährleistet.

Nach einer weiteren Ausführungsform der Erfindung können die Heizelemente auch selbst aus einem rund geformten Heizgewebe bestehen.

## Patentansprüche

1. Vorrichtung zum Temperieren einer Fluidleitung, wobei eine aus einem elastischen Material bestehende Mantelleitung (1, 21) vorgesehen ist mit einem Längsschlitz (5), an dem die Fluidleitung in einen Hohlraum (6) der Mantelleitung (1, 21) einsetzbar ist, sich in Längsrichtung der Mantelleitung (1, 21) mindestens ein Heizelement (11) erstreckt und in einem ersten Bereich und in einem zweiten Bereich der Mantelleitung (1, 21) jeweils ein Temperatursensor (14, 15) angeordnet ist, **dadurch gekennzeichnet, dass** die Mantelleitung (1, 21) zu beiden Seiten einer durch den Längsschlitz (5) verlaufenden Längsmittelebene (M) mindestens einen Umfangsabschnitt (7, 8, 22, 23) mit einer Mehrzahl von durchgehenden Längskanälen (9, 9', 10, 10', 13, 13', 13", 26, 26') und/oder teilweisen Längskanälen (13, 13', 13") aufweist, wobei in der Mehrzahl von Längskanälen (9, 9', 10, 10', 13, 13', 13", 26, 26') mindestens zwei Heizelemente (11) und dem mindestens ein Temperatursensor (14, 15) angeordnet sind und wobei die Längskanäle (9, 9', 10, 10', 13, 13', 13", 26, 26') und/oder teilweisen Längskanäle (13, 13', 13") symmetrisch zu der Längsmittelebene (M) der Mantelleitung (1, 21) und/oder zu einer Mittelebene des Umfangsabschnitts (7, 8, 22, 23) verlaufen.

2. Vorrichtung nach Anspruch 1, **dadurch gekennzeichnet, dass** der Umfangsabschnitt (7, 8, 22, 23) der Mantelleitung (1, 21.) zwei Längskanäle (9, 9', 10, 10') zur Aufnahme von jeweils einem Heizelement (11) aufweist, wobei die gedachte verbindungsfläche (V) zwischen den Längskanälen parallel zu der Längsmittelebene (M) der Mantelleitung (1, 21) verläuft.

3. Vorrichtung nach Anspruch 1 oder 2, **dadurch gekennzeichnet, dass** der Temperatursensor (14, 15) formschlüssig oder stoffschlüssig in dem Endbereich eines Hohlkanals (13, 13', 13", 26, 26') gehaltert ist und dass die Kontaktierleitungen des Temperatursensors (14, 15) einen wesentlich kleineren Querschnitt aufweisen als der Querschnitt des Hohlkanals (13, 13', 13'', 26, 26').

4. Vorrichtung nach Anspruch 3, **dadurch gekennzeichnet, dass** zwischen den Längskanälen (9, 9', 10, 10') für die Heizelemente (11) der Hohlkanal (13, 13', 13'', 26, 26') verläuft, in dem mindestens ein Temperatursensor (14, 15) und mindestens ein dem Temperatursensor (14, 15) mit einer Regeleinheit verbindenden Kontaktierleitung verläuft.

5. Vorrichtung nach Anspruch 4, **dadurch gekennzeichnet, dass** ein erster Umfangsabschnitt (22) und ein zweiter Umfangsabschnitt (23) der Mantelleitung (21) jeweils dem im Querschnitt als Langloch ausgebildeten Hohlkanal (26, 26') aufweisen, der sich zwischen zwei Längskanälen (9, 9', 10, 10') für die Aufnahme der Heizelemente (11) unter paralleler Anordnung seiner Längsmittelebene zu der Längsmittelebene (M) der Mantelleitung (21) erstreckt.

6. Vorrichtung nach einem der Ansprüche 1 bis 5, **dadurch gekennzeichnet, dass** das Heizelement (11) fest umschlossen in dem Umfangsabschnitt (22, 23) der Mantelleitung (1, 21) angeordnet ist.

7. Vorrichtung nach einem der Ansprüche 1 bis 6, **dadurch gekennzeichnet, dass** das Heizelement (11) als Einlegeteil von dem extrudierten Umfangsabschnitt (7, 8, 22, 23) der Mantelleitung (1, 21) umschlossen ist.

8. Vorrichtung nach einem der Ansprüche 1 bis 7, **dadurch gekennzeichnet, dass** die Mantelleitung (21) einen ersten Umfangsabschnitt (22) und einen zweiten Umfangsabschnitt (23) aufweist und im Querschnitt nierenförmig ausgebildet ist mit einer Längskerbe (25), die sich in einem Verbindungsbereich (24) zwischen dem ersten Umfangsabschnitt (22) und dem zweiten Umfangsabschnitt (23) erstreckt.

## Claims

1. Device for tempering of a tube containing fluid which comprises a jacket conduit made of a flexible material and provided with a longitudinal slot across which a fluid tube is insertable into a cavity of the jacket conduit, at least one heating element extending in longitudinal direction of said jacket conduit and one temperature sensor each arranged in a first and a second section of the jacket conduit, **characterized in that** at both sides of a longitudinal center plane (M) extending through the longitudinal slot (5) the jacket conduit (1, 21) is provided with at least one circumferential section (7, 8, 22, 23) having a plurality of continuous longitudinal channels (9, 9', 10, 10', 13, 13', 13", 26, 26') and/or intermittent longitudinal channels (13, 13', 13") in which at least two heating elements (11) and at least one temperature sensor (14, 15) are arranged and wherein said continuous longitudinal channels (9, 9', 10, 10', 13, 13', 13", 26, 26') and/or intermittent longitudinal channels (13, 13', 13") extend asymmetrically to said longitudinal center plane (M) of the jacket conduit (1, 21) and/or to a center plane of said circumferential section (7, 8, 22, 23).

2. Device according to Claim 1, **characterized in that** the circumferential section (7, 8, 22, 23) of the jacket conduit (1, 21) comprises two longitudinal channels (9, 9', 10, 10') each to receive one heating element (11), an imaginary junction area (V) between said longitudinal channels extending parallel to said longitudinal center plane (M) of the jacket conduit (1, 21).

3. Device according to Claim 1, or 2, **characterized in that** the temperature sensor (14, 15) is retained in the end portion of hollow channel (13, 13', 13", 26, 26') in a positive or tight fit mode and that the cross section of the contact lines of the temperature sensor (14, 15) is essentially smaller than that of the hollow channel (13, 13', 13", 26, 26').

4. Device according to Claim 3, **characterized in that** the hollow channel (13, 13', 13", 26, 26') extends between the longitudinal channels (9, 9', 10, 10') for the heating elements (11) in which hollow channel at least one temperature sensor (14, 15) and at least one contact line connecting said temperature sensor 14, 15) to a regulating unit are installed.

5. Device according to Claims 3 or 4, **characterized in that** a first circumferential section (22) and a second circumferential section (23) of the jacket conduit (21) each include the hollow channel (26, 26') having the cross section of a long hole and extending between two longitudinal channels (9, 9', 10, 10') to accommodate the heating elements (11) wherein the lon-gitudinal center plane extends parallel to the longitudinal center plane (M) of the jacket conduit (21).

6. Device according to any of the preceding Claims 1 to 5, **characterised in that** the heating element (11) is embedded in and tightly surrounded by the circumferential section (22, 23) of the jacket conduit (1, 21).

7. Device according to any of the preceding Claims 1 to 6, **characterized in that** an insert type heating element (11) is encapsulated by an extruded circumferential section (7, 8, 22, 23) of the jacket conduit (1, 21).

8. Device according to any of the preceding Claims 1 to 7, **characterized in that** the jacket conduit (21) comprises a first circumferential section (22) and a second circumferential section (23) having the cross-sectional shape of a kidney wherein a longitudinal notch (25) extending within a function area (24) between said first circumferential section (22) and said second circumferential section (23) is provided.

## Revendications

1. Dispositif pour tempérer un conduit pour fluide, une conduite sous gaine (1, 21), qui consiste en un matériau élastique, étant prévue, avec une fente longitudinale (5), à laquelle le conduit pour fluide peut être raccordé dans un espace vide (6) de la conduite sous gaine (1, 21), au moins un élément de chauffage (11) s'étendant dans la direction longitudinale de la conduite sous gaine (1, 21), et un capteur de température (14, 15) étant agencé dans une première section et, respectivement, dans une deuxième section de la conduite sous gaine (1, 21), **caractérisé en ce que** la conduite sous gaine (1, 21) présente, des deux côtés d'un plan médian longitudinal (M) passant par la fente longitudinale (5), au moins une section circonférentielle (7, 8, 22, 23) avec une pluralité de canaux longitudinaux continus (9, 9', 10, 10', 13, 13', 13", 26, 26') et / ou des canaux longitudinaux partiels (13, 13', 13"), au moins deux éléments de chauffage (11) et au moins un capteur de température (14, 15) étant disposés dans la pluralité des canaux longitudinaux (9, 9', 10, 10', 13, 13', 13", 26, 26'), et les canaux longitudinaux (9, 9', 10, 10', 13, 13', 13" , 26, 26') et / ou les canaux longitudinaux partiels (13, 13', 13") s'étendant symétriquement par rapport au plan médian longitudinal (M) de la conduite sous gaine (1, 21) et / ou d'un plan médian de la section circonférentielle (7, 8, 22, 23).

2. Dispositif selon, la revendication 1, **caractérisé en ce que** la section circonférentielle (7, 8, 22, 23) de la conduite sous gaine (1, 21) présente deux canaux longitudinaux continus (9, 9', 10, 10'), qui reçoivent chacun un élément de chauffage (11), la surface de liaison imaginaire (V) s'étendant entre les canaux longitudinaux, parallèlement au plan médian longitudinal (M) de la conduite sous gaine (1, 21).

3. Dispositif selon revendication 1 ou 2, **caractérisé en ce que** le capteur de température (14, 15) est maintenu par emboîtement ou par liaison de matière, dans la section finale d'un canal creux (13, 13', 13", 26, 26'), et que les conduites de connexion du capteur de température (14, 15) présente une section transversale considérablement plus petite que la section transversale du canal creux (13, 13', 13", 26, 26').

4. Dispositif selon la revendication 3, **caractérisé en ce qu'**entre les canaux longitudinaux (9, 9', 10, 10') pour les éléments de chauffage (11), s'étend le canal creux (13, 13', 13", 26, 26'), dans lequel est agencé au moins un capteur de température (14, 15) et au moins une conduite de connexion, qui relie ledit capteur de température (14, 15) à une unité de réglage.

5. Dispositif selon la revendication 3 ou 4, **caractérisé en ce qu'**une première section circonférentielle (22) et une deuxième section circonférentielle (23) de la conduite sous gaine (21) présentent chacune le canal creux (26, 26'), configuré, dans se section transversale, en tant que trou oblong, lequel s'étend entre deux canaux longitudinaux (9, 9', 10, 10') pour la réception des élément de chauffage (11), son plan médian longitudinal étant dispose parallèlement au plan médian longitudinal (M) de la conduite sous gaine (21).

6. Dispositif selon l'une des revendications 1 à 5, **caractérisé en ce que** l'élément de chauffage (11) est fixé en étant loge dans la section circonférentielle (22, 23) de la conduite sous gaine (1, 21).

7. Dispositif selon l'une des revendications 1 à 6, **caractérisé en ce que** l'élément de chauffage (11), en tant que pièce insérée, est entouré par la section circonférentielle, extrudée (7, 8, 22, 23) de la conduite sous gaine (1, 21).

8. Dispositif selon l'une des revendications 1 à 7, **caractérise en ce que** la conduite sous gaine (21) présente une premiers section circonférentielle (22) et une deuxième section circonférentielle (23) et est de conception réniforme avec une encoche longitudinale (25), qui s'étend dans la zone de liaison (24) entre la première section circonférentielle (22) et la deuxième section
